(19) Europäisches Patentamt
European Patent Office
Office européen des brevets

(11) **EP 3 179 233 B1**

(12) **EUROPEAN PATENT SPECIFICATION**

(45) Date of publication and mention
of the grant of the patent:
**23.10.2019 Bulletin 2019/43**

(21) Application number: **15829541.0**

(22) Date of filing: **31.07.2015**

(51) Int Cl.:
*G01N 21/61* (2006.01)   *G01N 21/3504* (2014.01)
*G01J 3/427* (2006.01)   *G01N 33/00* (2006.01)
*G01N 21/27* (2006.01)   *G01N 21/31* (2006.01)

(86) International application number:
**PCT/JP2015/071786**

(87) International publication number:
**WO 2016/021495 (11.02.2016 Gazette 2016/06)**

(54) **GAS DENSITY DETECTION DEVICE AND GAS DENSITY CALCULATION METHOD FOR GAS DENSITY DETECTION DEVICE**

GASDICHTENDETEKTIONSVORRICHTUNG UND GASDICHTENBERECHNUNGSVERFAHREN FÜR FÜR GASDICHTENDETEKTIONSVORRICHTUNG

DISPOSITIF DE DÉTECTION DE DENSITÉ DE GAZ, ET PROCÉDÉ DE CALCUL DE DENSITÉ DE GAZ POUR DISPOSITIF DE DÉTECTION DE DENSITÉ DE GAZ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.08.2014 JP 2014158519**

(43) Date of publication of application:
**14.06.2017 Bulletin 2017/24**

(73) Proprietor: **Murata Manufacturing Co., Ltd.
Nagaokakyo-shi, Kyoto 617-8555 (JP)**

(72) Inventors:
• **OGUSHI, Naoki
Nagaokakyo-shi
Kyoto 617-8555 (JP)**
• **ITO, Shigeo
Nagaokakyo-shi
Kyoto 617-8555 (JP)**

(74) Representative: **Reeve, Nicholas Edward
Reddie & Grose LLP
The White Chapel Building
10 Whitechapel High Street
London E1 8QS (GB)**

(56) References cited:
JP-A- H07 151 684    JP-A- 2004 309 391
JP-A- 2007 502 407    JP-A- 2014 074 629
JP-U- 3 004 459    US-A- 5 129 401

US-B1- 6 791 689

• Sgx: "Infrared Sensor Application Note 1 - A Background to Gas Sensing by Non-Dispersive Infrared (NDIR)", , 1 May 2007 (2007-05-01), pages 1-9, XP055459369, Retrieved from the Internet: URL:https://www.sgxsensortech.com/content/uploads/2014/08/AN1---A-Background-to-Gas-Sensing-by-Non-Dispersive-Infrared-NDIR.pd f [retrieved on 2018-03-14]
• Sgx ET AL: "Infrared Sensor Application Note 2 - Signal Processing for Infrared Gas Sensors", , 1 December 2010 (2010-12-01), pages 1-9, XP055459374, Retrieved from the Internet: URL:https://www.sgxsensortech.com/content/uploads/2014/08/AN2---Signal-Processing-fo r-Infrared-Gas-Sensors.pdf [retrieved on 2018-03-14]
• Sgx: "Infrared Sensor Application Note 3 - Design of Microcontroller Software for Infrared Gas Sensors", , 1 May 2007 (2007-05-01), pages 1-3, XP055459376, Retrieved from the Internet: URL:https://www.sgxsensortech.com/content/uploads/2014/08/AN3---Design-of-Microcontr oller-Software-for-Infrared-Gas-Sensors.pd f [retrieved on 2018-03-14]

- Sgx: "Infrared Sensor Application Note 4 - Design of Electronics for Infrared Gas Sensors", , 1 October 2009 (2009-10-01), pages 1-6, XP055459388, Retrieved from the Internet: URL:https://www.sgxsensortech.com/content/uploads/2014/08/AN4---Design-of-Electronics-for-Infrared-Gas-Sensors.pdf [retrieved on 2018-03-14]
- Sgx: "Infrared Sensor Application Note 5 - Determining Coefficients for Linearisation and Temperature Compensation", , 1 May 2009 (2009-05-01), pages 1-4, XP055459380, Retrieved from the Internet: URL:https://www.sgxsensortech.com/content/uploads/2014/08/AN5---Determining-Coefficients-for-Linearisation-and-Temperature-Compensation.pdf [retrieved on 2018-03-14]
- Welles: "Measuring carbon dioxide in the atmosphere", Micrometeorology in agricultural &mldr;,, 1 January 2005 (2005-01-01), XP055442236, Retrieved from the Internet: URL:https://dl.sciencesocieties.org/publications/books/abstracts/agronomymonogra/micrometeorolog/287 [retrieved on 2018-01-18]
- Anonymous: "Vaisala CARBOCAP(TM) Sensor zur Kohlendioxidmessung Box headline here", , 1 January 2012 (2012-01-01), pages 1-2, XP055458605, Retrieved from the Internet: URL:https://www.vaisala.com/sites/default/files/documents/CEN-TIA-G-Carbocap-Technology-description-B210780DE-C.pdf [retrieved on 2018-03-12]

## Description

Technical Field

The present invention relates to a gas concentration detection apparatus that is configured to calibrate a calibration curve used for the detection of a gas concentration in consideration of a change in output characteristics and a gas concentration calculation method for a gas concentration detection apparatus.

Background of the Invention

For example, there is a known technique for causing a nondispersive infrared (NDIR) gas concentration detection apparatus to detect the concentration of detection target gas on the basis of the infrared absorption amount of the gas. Japanese Unexamined Patent Application Publication No. 2013-76634 discloses such a gas concentration detection apparatus including a sensor unit and a sensor body detachable from the sensor unit. The infrared absorption amount of detection target gas is detected as the output characteristics of the sensor unit. A technique is also disclosed for calibrating the output characteristics of the sensor unit deviated from a value to be originally detected because of, for example, deterioration of a component by replacing only the sensor unit.

In the above-described gas concentration detection apparatus, an optical path is formed with a reflection member to increase the optical path length of infrared radiation. In such a gas concentration detection apparatus, after a long period of time has elapsed from the start of use, the reflection factor of infrared radiation from the inner wall of the reflection member forming the optical path is reduced because of a decrease in the output of a light source and the influences of dust and oxidation. Such a gas concentration detection apparatus converts the output characteristics of a concentration detection unit into the concentration of measurement target gas using a calibration curve stored in advance in a memory at the time of the start of use (for example, at the time of shipment). In a case where the above-described reduction in a reflection factor occurs, the concentration of measurement target gas calculated from the output characteristics of the concentration detection unit deviates from the actual concentration of measurement target gas. In the case of the technique disclosed in JP 2013-76634, it is necessary to manually replace a sensor unit to appropriately calibrate a change in output characteristics. This replacement is usually complicated. However, the appropriate calibration of the change in output characteristics cannot be achieved without the replacement of the sensor unit.

We have appreciated that it would be desirable to provide a gas concentration detection apparatus for easily calibrating output characteristics deviated from a value to be originally detected and a gas concentration calculation method for a gas concentration detection apparatus.

The SGX Sensortech Application Notes: "Infrared Sensor Application Note 1 - A Background to Gas Sensing by Non-Dispersive Infrared (NDIR)", "Infrared Sensor Application Note 2 - Signal Processing for Infrared Gas Sensors", "Infrared Sensor Application Note 3 - Design of Microcontroller Software for Infrared Gas Sensors", "Infrared Sensor Application Note 4 - Design of Electronics for Infrared Gas Sensors" and "Infrared Sensor Application Note 5 - Determining Coefficients for Linearisation and Temperature Compensation", to be found on the homepage of SGX Sensortech (www.sgxsensortech.com/content/uploads/2014/08) describe a method and apparatus to calibrate a non-dispersive infrared gas sensor.

## SUMMARY OF THE INVENTION

A gas concentration calculation method according to the present invention is for a gas concentration detection apparatus that includes a light source configured to emit infrared radiation, a light-receiving sensor configured to receive the infrared radiation emitted from the light source and output a first output value corresponding to an amount of light received, a reflection member that is provided between the light source and the light-receiving sensor and is configured to form an optical path of the infrared radiation, and a filter configured to, on the optical path, select and pass one of a first wavelength band in which infrared radiation absorptance of detection target gas is higher than that in another wavelength band and a second wavelength band in which infrared radiation absorptance of detection target gas is lower than that in the first wavelength band, and a temperature sensor configured to output a second output value corresponding to a temperature of the gas. The gas concentration calculation method includes the following four steps, a step of determining in advance the first output value obtained when the filter passes infrared radiation in the first wavelength band, a first calibration curve indicating a relationship between a concentration of the gas at a criterion temperature and a value obtained by normalizing the first output value with a criterion output value, and a second calibration curve indicating a relationship between the second output value in a criterion concentration and the criterion output value, a step of calculating a calibration factor used to calibrate the second calibration curve on the basis of the first output value obtained when the filter passes infrared radiation in the second wavelength band, a step of calibrating the second calibration curve on the basis of the calibration factor, and a step of calculating a concentration of the gas on the basis of the first calibration curve and the calibrated second calibration curve.

Thus, by calculating a calibration factor and calibrating the second calibration curve using the calculated calibration factor, the deterioration in detection accuracy of a gas concentration can be suppressed even in a case where the output characteristics of a gas concentration detection apparatus changes because of the deterioration of a component in the gas concentration detection apparatus.

The calibration factor calculating step preferably includes a step of calculating a conversion factor representing a ratio of a criterion output value, which is the first output value in the criterion concentration when the filter passes infrared radiation in the first wavelength band, to a reference output value, which is the first output value in the criterion concentration when the filter passes infrared radiation in the second wavelength band, and a step of calculating the calibration factor on the basis of the second output value, the reference output value, and the conversion factor.

Thus, since the calibration factor can be calculated on the basis of the second output value, the reference output value, and the conversion factor, the second calibration curve can be appropriately calibrated.

In the conversion factor calculating step, the conversion factor corresponding to the second output value in the criterion concentration is preferably calculated on the basis of a third calibration curve determined in advance.

Thus, since a conversion factor corresponding to the second output value can be calculated on the basis of the third calibration curve, the second calibration curve can be appropriately calibrated.

The calibration factor calculating step preferably includes a step of calculating a reference output value corresponding to the second output value obtained when the filter passes infrared radiation in the second wavelength band using a fourth calibration curve determined in advance and a step of calculating, as the calibration factor, a ratio of the reference output value to the first output value obtained when the filter passes infrared radiation in the second wavelength band.

Thus, since the ratio of the reference output value to the first output value obtained when the filter passes infrared radiation in the second wavelength band can be calculated as the calibration factor, the second calibration curve can be appropriately calibrated using the calculated calibration factor.

A gas concentration detection apparatus according to the present invention includes a light source configured to emit infrared radiation, a light-receiving sensor configured to receive the infrared radiation emitted from the light source and output a first output value corresponding to an amount of light received, a reflection member that is provided between the light source and the light-receiving sensor and is configured to form an optical path of the infrared radiation, a filter configured to, on the optical path, select and pass one of a first wavelength band in which infrared radiation absorptance of detection target gas is higher than that in another wavelength band and a second wavelength band in which infrared radiation absorptance of detection target gas is lower than that in the first wavelength band, a temperature sensor configured to output a second output value corresponding to a temperature of the gas, and a calculation unit configured to calculate a concentration of gas on the optical path. The first output value obtained when the filter passes infrared radiation in the first wavelength band, a first calibration curve indicating a relationship between a concentration of the gas at a criterion temperature and a value obtained by normalizing the first output value with a criterion output value, and a second calibration curve indicating a relationship between the second output value in a criterion concentration and the criterion output value are determined in advance. The calculation unit calculates a calibration factor used to calibrate the second calibration curve on the basis of the first output value obtained when the filter passes infrared radiation in the second wavelength band, calibrates the second calibration curve on the basis of the calibration factor, and calculates a concentration of the gas on the basis of the first calibration curve and the calibrated second calibration curve.

Thus, by calculating a calibration factor and calibrating the second calibration curve using the calculated calibration factor, the deterioration in detection accuracy of a gas concentration can be suppressed even in a case where the output characteristics of a gas concentration detection apparatus changes because of the deterioration of a component in the gas concentration detection apparatus.

The calculation units preferably calculates a conversion factor representing a ratio of a criterion output value, which is the first output value in the criterion concentration when the filter passes infrared radiation in the first wavelength band, to a reference output value, which is the first output value in the criterion concentration when the filter passes infrared radiation in the second wavelength band, and preferably calculates the calibration factor on the basis of the second output value, the reference output value, and the conversion factor.

Thus, since the calibration factor can be calculated on the basis of the second output value, the reference output value, and the conversion factor, the second calibration curve can be appropriately calibrated.

The calculation unit preferably calculates the conversion factor corresponding to the second output value in the criterion concentration on the basis of a third calibration curve determined in advance.

Thus, since a conversion factor corresponding to the second output value can be calculated on the basis of the third calibration curve, the second calibration curve can be appropriately calibrated.

The calculation unit preferably calculates a reference output value corresponding to the second output value obtained when the filter passes infrared radiation in the second wavelength band using a fourth calibration curve determined in advance, and preferably calculates, as the calibration factor, a ratio of the reference output value to the first output value obtained when the filter passes infrared radiation in the second wavelength band.

Thus, since the ratio of the reference output value to the first output value obtained when the filter passes infrared radiation in the second wavelength band can be calculated as the calibration factor, the second calibration curve can be appropriately calibrated using the calculated calibration factor.

The calculation unit preferably calculates the calibration factor each time a period of time determined in advance has elapsed.

Thus, by calculating the calibration factor each time a period of time determined in advance has elapsed, the output characteristics of the gas concentration detection apparatus can be appropriately calibrated.

The calculation unit preferably recalculates the calibration factor when a change in the second output value from a point in time when the calibration factor has been calculated is larger than or equal to a value determined in advance.

Thus, even in a case where the change in the second output value from a point in time when the calibration factor has been calculated is larger than or equal to a value determined in advance, the second calibration curve can be appropriately calibrated by recalculating the calibration factor.

When there is, in a history of previously calculated calibration factors, a calibration factor that satisfies conditions that the calibration factor has been obtained within a validity period of time and a difference between the second output value and the second output value obtained when the calibration factor has been calculated is smaller than a value determined in advance, the second calibration curve is preferably calibrated using the calibration factor.

Thus, since unnecessary calibration processing is not performed, the execution of calibration processing with good frequency can be achieved. For example, in a case where a switcher is an electric, power consumption can therefore be reduced.

The present invention can provide a gas concentration detection apparatus for easily calibrating output characteristics deviated from a value to be originally detected and a gas concentration calculation method for a gas concentration detection apparatus.

BRIEF DESCRIPTION OF DRAWINGS

Fig. 1 is a diagram illustrating the configuration of a gas concentration detection apparatus according to an embodiment of the present invention.
Fig. 2 is a plan view illustrating the configuration of a gas concentration detection apparatus according to an embodiment of the present invention.
Fig. 3 is a diagram illustrating the circuit configuration of a gas concentration detection apparatus according to an embodiment of the present invention.
Fig. 4 is a diagram describing a first calibration curve at a criterion temperature, a second calibration curve in a criterion concentration, and a third calibration curve used for the calibration of the second calibration curve.
Fig. 5 is a diagram illustrating the relationship between a filter temperature and a sensor output value.
Fig. 6 is a diagram illustrating the relationship between a temperature and a conversion factor.
Fig. 7 is a flowchart illustrating a calibration process.
Fig. 8 is a timing chart illustrating an execution model of a calibration process.

DETAILED DESCRIPTION OF EXAMPLE EMBODIMENTS

Embodiments of the present invention will be described below with reference to the drawings. In the following description, the same parts are denoted with the same reference signs. Their designations and functions are also the same. Therefore, detailed descriptions thereof will not be repeated.

First Embodiment

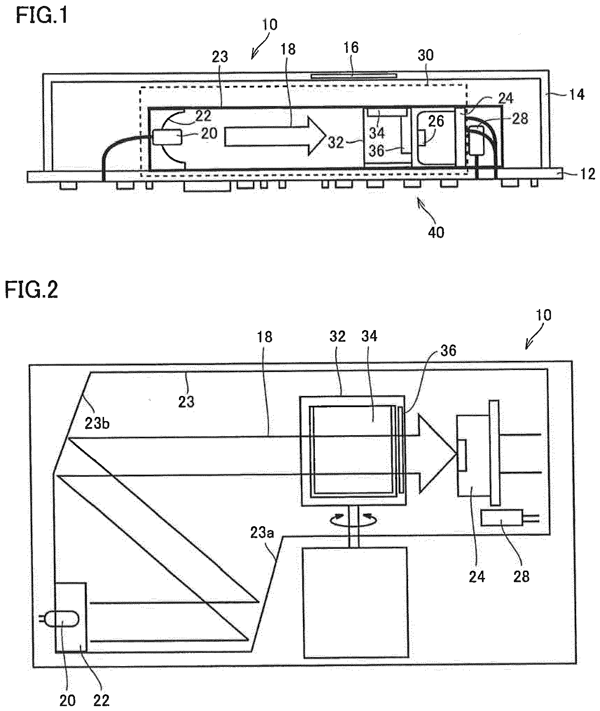

Fig. 1 is a diagram illustrating the configuration of a gas concentration detection apparatus 10 according to this embodiment.

The gas concentration detection apparatus 10 according to this embodiment is a nondispersive infrared (NDIR) gas sensor. Gas, the concentration of which is detected by the gas concentration detection apparatus 10 according to this embodiment, is carbon dioxide.

The gas concentration detection apparatus 10 is used to, for example, control the amount of ventilation on the basis of the concentration of carbon dioxide in a building energy management system (BEMS).

As illustrated in Fig. 1, the gas concentration detection apparatus 10 includes a concentration detection unit 30 for performing a gas concentration detection operation, a thermistor 28 that is a temperature detection unit for detecting the temperature of gas, and a driving circuit 40 for performing lighting control of a light source 20 and performing predetermined processing upon a concentration detection signal (a first output value) output from a pyroelectric sensor 24 to be described later and a temperature detection signal (a second output value) output from the thermistor 28. Components included in the concentration detection unit 30 and the thermistor 28 are provided at respective predetermined positions on one surface of a circuit board 12. Components included in the driving circuit 40 are provided at respective predetermined positions on the other surface of the circuit board 12.

The concentration detection unit 30 includes the light source 20, a holding table 22, a reflection member 23, the pyroelectric sensor 24 that is a light-receiving sensor, and a switcher 32 for switching between a plurality of types of filters.

The light source 20 is disposed apart from the pyroelectric sensor 24 by a predetermined distance. The light source 20 emits infrared radiation, so that an optical path 18 is formed between the light source 20 and the pyroelectric sensor 24. More specifically, infrared radiation emitted from the light source 20 is reflected by the reflection member 23, so that the optical path 18 is formed.

Fig. 2 is a plan view illustrating the configuration of a gas concentration detection apparatus according this embodiment. As illustrated in Fig. 2, the light source 20 and the pyroelectric sensor 24 do not face each other and are shifted in an up-and-down direction in Fig. 2. The reflection member 23 is placed inside a cover 14, and has a first wall surface 23a facing the light source 20 and a second wall surface 23b facing the pyroelectric sensor 24. The orientations (angles) of the first wall surface 23a and the second wall surface 23b are determined in advance so that the optical path 18, along which infrared radiation emitted from the light source 20 is directed to the pyroelectric sensor 24, is formed. Infrared radiation emitted from the light source 20 is partially reflected by the first wall surface 23a facing the light source 20 as represented by the optical path 18 illustrated in Fig. 2.

The infrared radiation reflected by the first wall surface 23a is partially directed to the second wall surface 23b and is then reflected by the second wall surface 23b. The infrared radiation reflected by the second wall surface 23b is partially directed to the pyroelectric sensor 24. Thus, the optical path 18 is formed.

In this embodiment, the light source 20 is, for example, a filament lamp. The light source 20 may be a light emitting diode (LED) on condition that it emits a wavelength including at least the wavelength of infrared radiation. The light source 20 is held by the holding table 22 fixed to the circuit board 12. The light source 20 is controlled to flash at predetermined intervals.

Referring back to Fig. 1, the holding table 22 has a semielliptical section that opens to the pyroelectric sensor 24. The semielliptical section has a mirror inner surface. Specifically, a part of an elliptical mirror is formed at the holding table 22. The light source 20 is placed at the focal position of the semielliptical section of the holding table 22. A part of the elliptical mirror is also formed at the reflection member 23. Accordingly, infrared radiation emitted from the light source 20 directly enters the pyroelectric sensor 24 after passing through the optical path 18, or enters the pyroelectric sensor 24 after being reflected by the mirror surfaces formed at the holding table 22 and the reflection member.

The pyroelectric sensor 24 is pyroelectric infrared sensor using bulk ceramics. At the pyroelectric sensor 24, an entrance window 26 for receiving infrared radiation emitted from the light source 20 is formed so that it faces the light source 20.

The switcher 32 is provided between the light source 20 and the pyroelectric sensor 24. The switcher 32 places a first band pass filter 34 or a second band pass filter 36 on the optical path between the light source 20 and the pyroelectric sensor in accordance with a control signal transmitted from a switching driving circuit 48 to be described later. The switcher 32 switches between the first band pass filter 34 and the second band pass filter 36 using, for example, an actuator such as a motor. The first band pass filter 34 corresponds to a first filter in claims, and the second band pass filter 36 corresponds to a second filter in claims.

The first band pass filter 34 passes infrared radiation in a first wavelength band including a wavelength near 4.26 $\mu$m at which the absorptance of carbon dioxide is high. In a case where the switcher 32 places the first band pass filter 34 on the optical path, the pyroelectric sensor 24 receives infrared radiation in the first wavelength band that is part of infrared radiation emitted from the light source 20. A value output from the pyroelectric sensor 24 is converted into the concentration of carbon dioxide.

The second band pass filter 36 passes infrared radiation in a second wavelength band that is different from the first wavelength band and includes a wavelength (for example, 3.9 $\mu$m) at which the absorptance of detection target gas is low. In a case where the switcher 32 places the second band pass filter 36 on the optical path, the pyroelectric sensor 24 receives infrared radiation in the second wavelength band that is part of infrared radiation emitted from the light source 20.

The thermistor 28 is disposed near the pyroelectric sensor 24 and is fixed to the circuit board 12. Upon the application of a voltage from the driving circuit 40, a constant current flows through the thermistor 28. The driving circuit 40 detects a voltage generated when the constant current flows through the thermistor 28 as an output voltage.

The cover 14 covers the components in the concentration detection unit 30 and the thermistor 28 and is fixed to the circuit board 12. The cover 14 has an opening 16 from which gas is externally input or emitted. At the opening 16, an air filter is disposed.

The gas concentration detection apparatus 10 detects the concentration of carbon dioxide in a state where gas is input into the inside of the cover 14 from the opening 16. When infrared radiation is emitted from the light source 20 to the pyroelectric sensor 24, the pyroelectric sensor 24 receives the emitted infrared radiation and outputs a voltage based on the amount of infrared radiation received.

In a case where the first band pass filter 34 is placed on the optical path, a voltage output from the pyroelectric sensor 24 changes depending on the concentration of carbon dioxide on the optical path 18. The reason for this is that part of infrared radiation emitted from the light source 20, which is in the first wavelength band and is to pass through the first band pass filter 34, is absorbed by carbon dioxide on the optical path 18. The amount of infrared radiation that is emitted from the light source 20 and reaches the pyroelectric sensor 24 via the first band pass filter 34 therefore changes (the Lambert-Beer law).

In a case where the second band pass filter 36 is placed on the optical path, a voltage output from the pyroelectric sensor 24 does not change depending on the concentration of carbon dioxide on the optical path 18. The reason for this is that part of infrared radiation emitted from the light source 20, which is in the second wavelength band and is to pass through the second band pass filter, is poorly absorbed by carbon dioxide or another gas.

On the other hand, irrespective of which of the first band pass filter 34 and the second band pass filter 36 is placed on the optical path, a voltage output from the pyroelectric sensor 24 changes in accordance with a temperature.

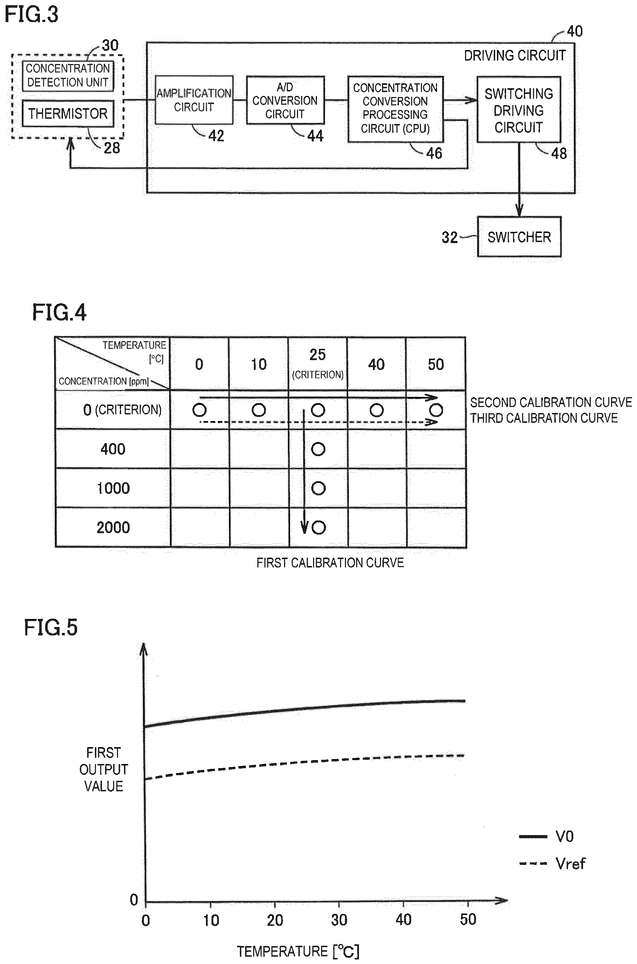

Fig. 3 is a diagram illustrating the circuit configuration of the gas concentration detection apparatus 10 according to this embodiment. As illustrated in Fig. 3, the driving circuit 40 includes an amplification circuit 42, an A/D conversion circuit 44, a concentration conversion processing circuit 46, and the switching driving circuit 48. The concentration conversion processing circuit 46 corresponds to a calculation unit in claims.

The amplification circuit 42 is, for example, an amplifier, and amplifies the strength of a concentration detection signal (the first output value) output from the pyroelectric sensor 24 and the strength of a temperature detection signal (the second output value) output from the thermistor 28.

The A/D conversion circuit 44 converts an analog signal, which has been subjected to signal strength amplification in the amplification circuit 42, into a digital signal. The amplification of a signal strength and the conversion of an analog signal into a digital signal may be performed using known techniques.

The concentration conversion processing circuit 46 performs predetermined processing upon a digital signal converted by the A/D conversion circuit 44 to calculate a carbon dioxide concentration $C_x$ in gas input into the inside of the cover 14. In this embodiment, the concentration conversion processing circuit 46 is realized by, for example, a central processing unit (CPU). A CPU executes a program stored in a memory (not illustrated) to perform predetermined computation processing and predetermined control processing. For example, a CPU performs not only computation processing for calculating the concentration of carbon dioxide but also control processing for turning on the light source 20, control processing for applying a voltage to the thermistor 28, and control processing for operating the switcher 32 to place either the first band pass filter 34 or the second band pass filter 36 on the optical path between the light source 20 and the pyroelectric sensor 24. A CPU outputs a driving instruction to the switching driving circuit 48 when operating the switcher 32. Upon receiving the driving instruction from the CPU, the switching driving circuit 48 generates a control signal and outputs the control signal to the switcher 32.

In this embodiment, the gas concentration detection apparatus 10 detects the concentration of carbon dioxide using the following procedure. It is assumed that the switcher 32 selects the first band pass filter 34 (that is, the first band pass filter 34 is placed on the optical path).

(1) Acquiring a temperature detection signal from the thermistor 28. (2) Turning on the light source 20. (3) Acquiring an output value V of the pyroelectric sensor 24. (4) Performing predetermined signal processing upon the output value V of the pyroelectric sensor 24.

The output value V of the pyroelectric sensor 24 corresponds to the first output value according to the present invention. A temperature detection signal output from the thermistor 28 corresponds to the second output value according to the present invention.

The predetermined signal processing includes, for example, processing for removing noise from the output waveform of the pyroelectric sensor 24 using the method of moving averages, processing for causing the amplification circuit 42 to amplify a signal strength, and processing for causing the A/D conversion circuit 44 to obtain digital data by conversion. These pieces of processing are also performed upon a temperature detection signal. On the basis of a temperature detection signal, a thermistor temperature Th[K] is calculated. The calculation of the thermistor temperature Th may be performed in (1) in the above-described procedure.

(5) Calculating the carbon dioxide concentration $C_x$ on the basis of the thermistor temperature Th and the output value V of the pyroelectric sensor 24. (6) Turning off the light source 20. The gas concentration detection apparatus 10 performs the procedure from (1) to (6), for example, at regular time intervals.

A method of causing the gas concentration detection apparatus 10 according to this embodiment to calculate the carbon dioxide concentration $C_x$ on the basis of the temperature Th of the thermistor 28 and the output value V of the pyroelectric sensor 24.

The concentration conversion processing circuit 46 calculates the concentration of carbon dioxide on the basis of the output value V of the pyroelectric sensor 24 and the first and second calibration curves acquired in advance.

Data of the first calibration curve and data of the second calibration curve are acquired in advance at the time of manufacture of the gas concentration detection apparatus 10 and are stored in a storage medium such as a memory in the driving circuit 40.

The first calibration curve indicates the relationship between the carbon dioxide concentration $C_x$ and the output value V of the pyroelectric sensor 24 at a criterion temperature (25°C) determined in advance. More specifically, the first calibration curve indicates the relationship between a value ($V/V_0$) obtained by normalizing the output value V of the pyroelectric sensor 24 with a criterion output value $V_0$ and the carbon dioxide concentration $C_x$. A first calibration curve is set by deriving an approximation of a predetermined order on the basis of the output values of the pyroelectric sensor 24, which have been acquired in advance, corresponding one-to-one to a plurality of concentrations of carbon dioxide at a criterion temperature.

This criterion output value $V_0$ is the output value of the pyroelectric sensor 24 corresponding to the thermistor temperature Th in a case where the concentration of carbon dioxide is a criterion concentration (e.g. 0 ppm) determined in advance. The criterion output value $V_0$ is calculated on the basis of the thermistor temperature Th using the second calibration curve to be described later.

In this embodiment, the first calibration curve is represented by the following equation.

$$C_x \text{ (the concentration of carbon dioxide)} = f(V/V_0) \cdots \text{(Equation 1)}$$

In the above-described Equation 1, f represents a function of a predetermined order, and may be, for example, a quadratic function or a cubic function. In a case where f represents an Nth-order function, the gas concentration $C_x$ is represented by the following equation.

$$C_x = a_1 \times (V/V_0)^N + a_2 \times (V/V_0)^{N-1} + \cdots + a_{N+1} \cdots \text{(Equation 2)}$$

In the above-described Equation 2, $a_1$ to $a_{N+1}$ are calculated on the basis of the combination of each of a plurality of types of concentrations of carbon dioxide and the output value of the pyroelectric sensor 24 which has been acquired in advance by, for example, experiments at a criterion temperature. For example, as illustrated in Fig. 4, the output values of the pyroelectric sensor 24 corresponding one-to-one to a plurality of concentrations of carbon dioxide (for example, 0 ppm, 400 ppm, 1000 ppm, and 2000 ppm) determined in advance at a criterion temperature are acquired in advance by, for example, experiments, and $a_1$ to $a_{N+1}$ are calculated on the basis of the acquired output values.

The above-described Equation 2 and the calculated values of $a_1$ to $a_{N+1}$ are stored in a storage medium such as a memory provided in the driving circuit 40.

The thermistor temperature Th is calculated using the following equation.

$$Th = 1/[1/Th_{25} + 1/B \times \ln\{V_{th}/(V_{cc} - V_{th})\}] \cdots \text{(Equation 3)}$$

In the above-described Equation 3, B represents a constant, $V_{th}$ represents the output voltage of the thermistor 28, $V_{cc}$ represents a voltage applied from the driving circuit 40 to the thermistor 28, and $Th_{25}$ represents a thermistor temperature [K] at 25°C. The above-described Equation 3 and the constant B are stored in a storage medium such as a memory provided in the driving circuit 40.

The concentration conversion processing circuit 46 calculates the criterion output value $V_0$ using the second calibration curve. The second calibration curve indicates the relationship between the thermistor temperature Th and the criterion output value $V_0$ in a criterion concentration (for example, 0 ppm) determined in advance. A second calibration curve is set by deriving an approximation of a predetermined order on the basis of the output values of the pyroelectric sensor 24, which have been acquired in advance, corresponding one-to-one to a plurality of temperatures of carbon dioxide in a criterion concentration in a case where the first band pass filter 34 is selected.

In this embodiment, the second calibration curve is represented by the following equation.

$$V_0 = g(Th) \cdots \text{(Equation 4)}$$

In the above-described Equation 4, g represents a function of a predetermined order. In a case where g represents an Nth-order function, the criterion output value $V_0$ is represented by the following equation.

$$V_0 = b_1 \times Th^N + b_2 \times Th^{N-1} + \cdots + b_{N+1} \text{ (Equation 5)}$$

In the above-described Equation 5, $b_1$ to $b_{N+1}$ are calculated on the basis of the combination of each of a plurality of types of temperatures (the thermistor temperatures Th) and the output value of the pyroelectric sensor 24 which has been acquired in advance by, for example, experiments in a criterion concentration in a case where the first band pass filter 34 is selected.

As illustrated in Fig. 4, the output values of the pyroelectric sensor 24 corresponding one-to-one to a plurality of temperatures (0°C, 10°C, 25°C, 40°C, and 50°C) in a criterion concentration are acquired in advance by, for example, experiments, and $b_1$ to $b_{N+1}$ are calculated on the basis of the acquired output values.

The above-described Equation 5 and the calculated values of $b_1$ to $b_{N+1}$ are stored in a storage medium such as a memory provided in the driving circuit 40.

In this embodiment, the concentration conversion processing circuit 46 calibrates the second calibration curve. More specifically, a third calibration curve represented by a broken-line arrow in Fig. 4 is acquired and the second calibration curve is calibrated using the third calibration curve. The third calibration curve is calculated on the basis of the thermistor temperature Th and the ratio (hereinafter referred to as a conversion factor C) of an concentration output value (criterion output value $V_0$) in a criterion concentration when the first band pass filter 34 is selected to a concentration output value (hereinafter referred to as a reference output value $V_{ref}$) in the criterion concentration when the second band pass filter 36 is selected. A third calibration curve is set by deriving an approximation of a predetermined order on the basis of the output values of the pyroelectric sensor 24, which have been acquired in advance, corresponding one-to-one to a plurality of temperatures of carbon dioxide in a criterion concentration.

In this embodiment, the third calibration curve is represented by the following equation.

$$C = V_0/V_{ref} = h(Th) \cdots \text{ (Equation 6)}$$

Since the criterion output value $V_0$ and the reference output value $V_{ref}$ are functions in which Th serves as a variable, they can be represented as a single function in which Th serves as a variable as expressed by the above-described Equation 6. In Equation 6, h represents a function of a predetermined order. For example, in a case where h is an Nth-order function, the conversion factor C is represented by the following equation.

$$C = c_1 \times Th^N + c_2 \times Th^{N-1} + \cdots + c_{N+1} \cdots \text{ (Equation 7)}$$

In the above-described Equation 7, $c_1$ to $c_{N+1}$ are calculated on the basis of the combination of each of a plurality of types of temperatures (the thermistor temperatures Th), the output value (criterion output value) of the pyroelectric sensor 24 when the first band pass filter 34 is selected, and the output value (reference output value) of the pyroelectric sensor 24 when the second band pass filter 36 is selected which has been acquired in advance in a criterion concentration by, for example, experiments.

For example, as illustrated in Fig. 5, the criterion output values $V_0$ and the reference output values $V_{ref}$ corresponding one-to-one to a plurality of temperatures (0°C, 10°C, 25°C, 40°C, and 50°C) in a criterion concentration are acquired in advance by, for example, experiments, and $c_1$ to $c_{N+1}$ are calculated on the basis of the acquired output values. In Fig. 5, a vertical axis represents the first output value of the pyroelectric sensor 24 and a horizontal axis represents a temperature.

The above-described Equation 7 and the calculated values of $c_1$ to $c_{N+1}$ are stored in a storage medium such as a memory provided in the driving circuit 40.

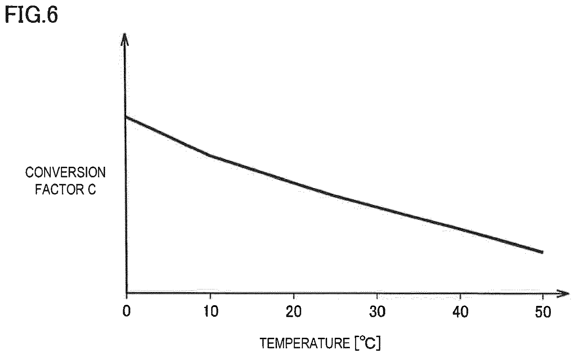

As illustrated in Fig. 6, the higher the temperature, the smaller the conversion factor C represented by the third calibration curve. In Fig. 6, a vertical axis represents the conversion factor C and a horizontal axis represents a temperature.

The concentration conversion processing circuit 46 calculates a factor (hereinafter referred to as a calibration factor R) used to calibrate the second calibration curve using the third calibration curve.

The calibration factor R is defined by the following equation in which $V_0$ represents a factory-default criterion output value and $V_0$' represents a criterion output value at the time of calibration.

$$R = V_0'/V_0 \cdots \text{(Equation 8)}$$

The value of $V_0$ is calculated by the equation of $V_0 = g(Th_{New})$ using the second calibration curve and a thermistor temperature $Th_{New}$ at the time of calibration. On the other hand, $V_0'$ is a concentration output value in a criterion concentration at the time of calibration in a case where the first band pass filter 34 is selected. In an actual environment at the time of calibration, since a criterion concentration is not always achieved, $V_0'$ is calculated by the equation of $V_0' = V_{ref} \times C$. The above-described Equation 8 is therefore rewritten as $R = V_{ref} \times C/g(Th_{New})$. Since the conversion factor C is defined by the above-described Equation 6, the calibration factor R can be calculated irrespective of a gas concentration at the time of calibration.

In a case where the calibration factor R is calculated, the concentration conversion processing circuit 46 calibrates the second calibration curve using the calculated calibration factor R. Specifically, the following equation is used as a post-calibration second calibration curve.

$$V_0 = R \times g(Th) \cdots \text{(Equation 9)}$$

After calibration, the concentration conversion processing circuit 46 uses the above-described Equation 9 instead of the above-described Equation 4 to calculate the gas concentration $C_x$. That is, the concentration conversion processing circuit 46 calculates the criterion output value $V_0$ using the post-calibration second calibration curve represented by the above-described Equation 9 and calculates the gas concentration $C_x$ using the calculated criterion output value $V_0$ and the first calibration curve.

The timing of execution of calibration processing by the concentration conversion processing circuit 46 will be described below.

The concentration conversion processing circuit 46 performs first calibration processing, for example, after a predetermined period of time has elapsed from first use. Before performing the first calibration processing, the concentration conversion processing circuit 46 calculates the criterion output value $V_0$ using the factory-default second calibration curve represented by the above-described Equation 4. After performing the first calibration processing, the concentration conversion processing circuit 46 calculates the criterion output value $V_0$ using the post-calibration second calibration curve represented by the above-described Equation 9. After performing the first calibration processing, the concentration conversion processing circuit 46 performs calibration processing each time a predetermined period of time has elapsed.

Even before a predetermined period of time has elapsed from the execution of the first calibration processing, the concentration conversion processing circuit 46 performs calibration processing in a case where there is a change (increase or decrease) in environmental temperature from a temperature at the time of execution of the last calibration processing which is larger than or equal to a threshold value. The predetermined period of time is, for example, 24 hours. The threshold value is, for example, $\pm5°C$ from a temperature at the time of execution of the last calibration processing.

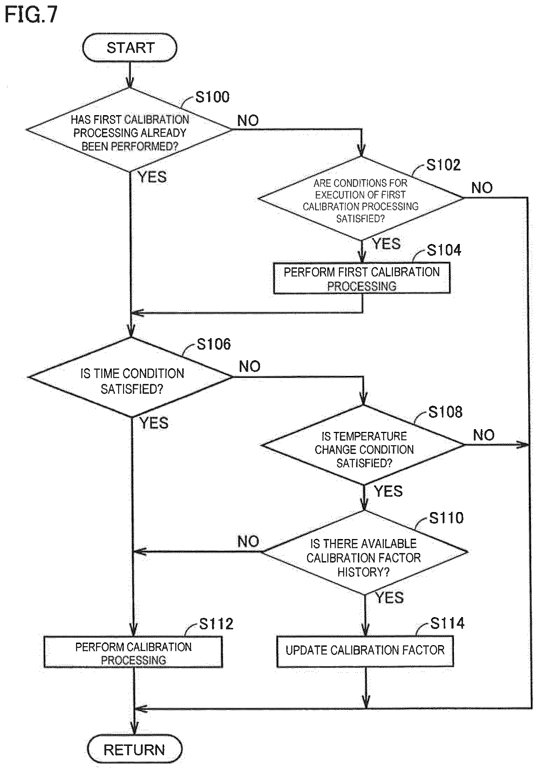

In this embodiment, the concentration conversion processing circuit 46 performs a calibration process illustrated in Fig. 7.

Specifically, in step (hereinafter referred to as "S") 100, the concentration conversion processing circuit 46 determines whether the first calibration processing has already been performed. For example, in a case where a flag indicating that the first calibration processing has already been performed is ON, the concentration conversion processing circuit 46 determines that the first calibration processing has already been performed. It is determined that the first calibration processing has already been performed (YES in S100), the process proceeds to S106. If not (NO in S100), the process proceeds to S102.

In S102, the concentration conversion processing circuit 46 determines whether conditions for execution of the first calibration processing are satisfied. Conditions for execution of the first calibration processing include, for example, a condition that a period of time determined in advance has elapsed from first use, but may include another condition (for example, a temperature condition) in addition to or instead of this condition. In a case where it is determined that conditions for execution of the first calibration processing are satisfied (YES in S102), the process proceeds to S104. If not (NO in S102), the process ends.

In S104, the concentration conversion processing circuit 46 performs the first calibration processing. The calibration processing has been described in detail above, and the detailed description thereof will not therefore be repeated. At that time, the concentration conversion processing circuit 46 sets a flag indicating that the first calibration processing has already been performed to ON. After performing the first calibration processing, the concentration conversion processing circuit 46 calculates the gas concentration $C_x$ using the post-calibration second calibration curve (represented by the above-described Equation 9).

In S106, the concentration conversion processing circuit 46 determines whether a time condition is satisfied. More specifically, the concentration conversion processing circuit 46 determines whether a time condition that a period of time determined in advance has elapsed from the execution of the first calibration processing or the satisfaction of the last time condition is satisfied. In a case where it is determined that a time condition is satisfied (YES in S106), the process proceeds to S112. If not (NO in S106), the process proceeds to S108.

In S108, the concentration conversion processing circuit 46 determines whether a temperature change condition is satisfied. The concentration conversion processing circuit 46 determines that a temperature change condition is satisfied in a case where the difference between a temperature at which the last calibration processing has been performed and a current temperature is larger than or equal to a threshold value. In a case where it is determined that a temperature change condition is satisfied (YES in S108), the process proceeds to S110. If not (NO in S108), the process ends.

In S110, the concentration conversion processing circuit 46 determines whether there is the available calibration factor R in the history of the previously calculated calibration factors R. More specifically, the concentration conversion processing circuit 46 determines whether, in the history of the previously calculated calibration factors R, there is a calibration factor R satisfying conditions that a current temperature is in a predetermined temperature range from a temperature at which the calibration factor R has been calculated (a range from an upper limit obtained by adding a predetermined value to the temperature at the time of calculation to a lower limit obtained by subtracting the predetermined value from the temperature at the time of calculation) and a period of time elapsed from the calculation of the calibration factor R is within a validity period of time. In a case where it is determined that there is the available calibration factor R in the history of the previously calculated calibration factors R (YES in S110), the process proceeds to S114. If not (NO in S110), the process proceeds to S112.

In S112, the concentration conversion processing circuit 46 performs the calibration processing. In S114, the concentration conversion processing circuit 46 updates the calibration factor R using the calibration factor R determined to be available. In a case where there are calibration factors R determined to be available, the concentration conversion processing circuit 46 may update the calibration factor R using the immediately preceding calibration factor R or the calibration factor R calculated at a temperature closest to a current temperature.

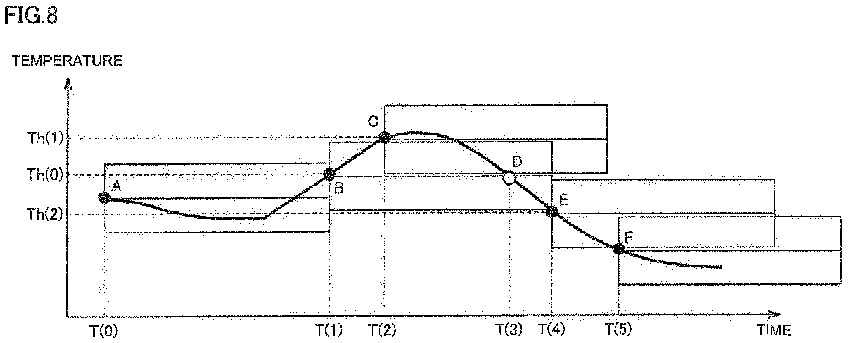

An exemplary operation of the gas concentration detection apparatus 10 in a case where the calibration process illustrated in Fig. 7 is performed will be described with reference to Fig. 8. Referring to Fig. 8, points A to F are updating points of the calibration factor R. Rectangular regions that include respective points A to F as median values of left ends represent validity ranges of the calibration factor R calculated at corresponding points.

As a time T(0), in a case where the first calibration processing has not been performed (NO in S100) and conditions for execution of the first calibration processing are satisfied (YES in S102), the first calibration processing is performed (S104) (a point A).

At a time T(1), in a case where the first calibration processing has already been performed (YES in S100) and a period of time determined in advance has elapsed from the execution of the last calibration processing (YES in S106), the calibration processing is performed (S112) (a point B).

At a time T(2) before a period of time determined in advance has elapsed from the time T(1) (NO in S106), in a case where the difference between a temperature Th(0) at the time of execution of the last calibration processing (the time T(1)) and a current temperature Th is larger than or equal to a threshold value (YES in S108) and there is no calibration factor R in the history of the previously calculated calibration factors R (NO in S110), the calibration processing is performed (S112) (a point C).

After that, an environmental temperature decreases. At a time T(3) before a period of time determined in advance has elapsed from the time T(1), in a case where the difference between a temperature Th(1) at the time of execution of the last calibration processing (the time T(2)) and the current temperature Th is larger than or equal to the threshold value (YES in S108), it is determined whether there is the available calibration factor R in the history of the previously calculated calibration factors R (S110). In a case where a current temperature and a current time are within a validity range of the calibration factor R calculated at the time T(1) (the point B), it is determined that there is the available calibration factor R (YES in S110) and the calibration factor R is updated using the calibration factor R calculated at the point B (S114) (a point D).

At a time T(4) at which a period of time determined in advance has elapsed from the time T(1) (YES in S106), the calibration processing is performed (S112) (a point E). After that, an environmental temperature further decreases. At a time T(5) before a period of time determined in advance has elapsed from the time T(4), in a case where the difference between a temperature Th(2) at the time of execution of the last calibration processing (the time T(4)) and a current temperature Th is larger than or equal to the threshold value (YES in S108) and there is no calibration factor R in the history of the previously calculated calibration factors R (NO in S110), the calibration processing is performed (S112) (a point F).

Thus, even in a case where the output characteristics of the gas concentration detection apparatus 10 change because of, for example, the deterioration of a component such as the light source 20, the gas concentration detection apparatus 10 according to this embodiment calculates the calibration factor R using the third calibration curve and the above-described Equation 8 and can appropriately calibrate the second calibration curve using the calculated calibration factor R. The deterioration in detection accuracy of a gas concentration can therefore be suppressed. It is therefore possible to provide a gas concentration detection apparatus that appropriately calibrates a changed output characteristics and a gas concentration calculation method for a gas concentration detection apparatus.

Furthermore, by calculating the calibration factor R using the third calibration curve indicating the relationship between the conversion factor C and the thermistor temperature Th, the calibration factor R with which the second calibration curve can be appropriately calibrated irrespective of a concentration at the time of calibration can be calculated.

Furthermore, by calculating the calibration factor R each time a period of time determined in advance has elapsed, the output characteristics of the gas concentration detection apparatus 10 can be appropriately calibrated.

Furthermore, in a case where a temperature significantly changes, the second calibration curve cannot be appropriately calibrated using the calibration factor R calculated before the change in some cases. By performing the calibration processing, the calibration factor R corresponding to the changed temperature can be calculated. The second calibration curve can therefore be appropriately calibrated.

Furthermore, by using the history of calibration factors, the need to recalculate a calibration factor is eliminated. The execution of unnecessary calibration processing can therefore be suppressed, and the execution of calibration processing with good frequency can be achieved. The reduction in the execution frequency of the calibration processing leads to the reduction in power consumption at the time of switching by the switcher 32.

Modifications will be described below.

In the above-described embodiment, concentration detection target gas of the gas concentration detection apparatus 10 is carbon dioxide. However, detection target gas is not limited to carbon dioxide, and may be, for example, carbon monoxide, $CH_4$, or $NO_x$. In a case where a concentration detection target is gas other than carbon dioxide, the first wavelength band is selected on the basis of a wavelength band based on the type of the concentration detection target gas (that is, a wavelength at which the absorptance of the concentration detection target gas is high).

In the above-described embodiment, a switcher mechanically switches between filters by placing a first band pass filter or a second band pass filter on an optical path between a light source and a pyroelectric sensor in accordance with a control signal transmitted from a switching driving circuit. The number of filters is not limited two, and any filter capable of, on an optical path, selecting and passing one of a first wavelength band in which the infrared radiation absorptance of detection target gas is higher than that in another wavelength band and a second wavelength band in which the infrared radiation absorptance of detection target gas is lower than that in the first wavelength band may be used. For example, a Fabry-Perot filter for electrically switching between filters may be placed on an optical path between a light source and a pyroelectric sensor instead of the first band pass filter and the second band pass filter.

Instead of a plurality of types of temperatures (the thermistor temperatures Th) obtained in advance in a criterion concentration by experiments, the gas concentration detection apparatus 10 may use voltage values (thermistor output voltages $V_{th}$) at a plurality of types of temperatures obtained in advance in a criterion concentration by experiments. That is, the criterion output value $V_0$ may be calculated on the basis of the thermistor output voltage $V_{th}$. In the above-described Equations 4 and 5, Th may be therefore replaced with $V_{th}$.

In this embodiment, the conversion factor C has temperature dependence in accordance with the third calibration curve. However, in a case where temperature dependence is small, the conversion factor C may be a value determined in advance.

The configuration of the gas concentration detection apparatus 10 illustrated in Fig. 1 is illustrative only, and the configuration of the gas concentration detection apparatus 10 is not limited to the configuration illustrated in Fig. 1.

The circuit configuration of the gas concentration detection apparatus 10 illustrated in Fig. 3 is illustrative only, and the circuit configuration of the gas concentration detection apparatus 10 is not limited to the circuit configuration illustrated in Fig. 3.

A criterion temperature determined in advance is 25°C in this embodiment, but may be another temperature.

In the above-described embodiment, the output values of the pyroelectric sensor 24 corresponding to a plurality of temperatures (0°C, 10°C, 25°C, 40°C, and 50°C) in a criterion concentration are obtained in advance by, for example, experiments. Furthermore, in the above-described embodiment, the criterion output values V0 and the reference output values $V_{ref}$ corresponding to a plurality of temperatures (0°C, 10°C, 25°C, 40°C, and 50°C) in a criterion concentration are obtained in advance by, for example, experiments. However, a plurality of temperatures may include another temperature other than the above-described temperatures.

In this embodiment, in the calibration processing, the second calibration curve is calibrated on the basis of the change in output characteristics from the factory-default second calibration curve. However, the second calibration curve may be calibrated on the basis of the change in output characteristics from the second calibration curve obtained after the last calibration processing.

In this embodiment, the reference output value $V_{ref}$ is a concentration output value in a criterion concentration in a case where the second band pass filter 36 is selected. However, since the reference output value $V_{ref}$ is not affected by the absorption of detection target gas, there is no need to set the reference output value $V_{ref}$ on the basis of a concentration output value in a criterion concentration. The reference output value $V_{ref}$ may be set irrespective of concentration. The above-described modifications may be implemented in whole or in part.

Second Embodiment

A gas concentration detection apparatus according to the second embodiment will be described. A gas concentration detection apparatus according to this embodiment is the same as the gas concentration detection apparatus 10 according to the first embodiment except for the operation of the concentration conversion processing circuit 46. The same parts are denoted with the same reference signs. Their functions are also the same. Therefore, detailed descriptions thereof will not be repeated.

In the above-described first embodiment, the second calibration curve is calibrated using the third calibration curve indicating the relationship between a conversion factor and a temperature output value. In this embodiment, a fourth calibration curve is additionally provided, and the second calibration curve is calibrated using the fourth calibration curve instead of the third calibration curve.

The fourth calibration curve indicates the relationship between the thermistor temperature Th and a reference output value $V_{0r}$. The fourth calibration curve is set by deriving an approximation of a predetermined order on the basis of the output values of the pyroelectric sensor 24, which have been acquired in advance, corresponding one-to-one to a plurality of temperatures of carbon dioxide in a case where the second band pass filter 36 is selected.

In this embodiment, the fourth calibration curve is represented by the following equation.

$$V_{0r} = g_{ref}(Th) \cdots \text{(Equation 10)}$$

In the above-described Equation 10, $g_{ref}$ represents a function of a predetermined order. For example, in a case where $g_{ref}$ is an Nth-order function, the reference output value $V_{0r}$ is represented by the following equation.

$$V_{0r} = d_1 \times Th^N + d_2 \times Th^{N-1} + \cdots + d_{N+1} \cdots \text{(Equation 11)}$$

In the above-described Equation 11, $d_1$ to $d_{N+1}$ are calculated on the basis of the combination of each of a plurality of types of temperatures (the thermistor temperatures Th) and the output value of the pyroelectric sensor 24 which has been acquired in advance by, for example, experiments in a case where the second band pass filter 36 is selected.

For example, the output values of the pyroelectric sensor 24 corresponding one-to-one to a plurality of temperatures (0°C, 10°C, 25°C, 40°C, and 50°C) are obtained in advance by, for example, experiments, and $d_1$ to $d_{N+1}$ are calculated on the basis of the obtained output values.

The above-described Equation 11 and the calculated values of $d_1$ to $d_{N+1}$ are stored in a storage medium such as a memory provided in the driving circuit 40.

In this embodiment, the concentration conversion processing circuit 46 calculates the calibration factor R using the fourth calibration curve.

In the above-described first embodiment, the calibration factor R is calculated using the above-described Equation 8. In this embodiment, the calibration factor R is calculated using the following equation in which $V_{0r}$ represents a factory-default reference output value and $V_{0r}'$ represents a reference output value at the time of calibration.

$$R = V_{0r}/V_{0r}' \cdots \text{(Equation 12)}$$

The calibration factor R calculated using the above-described Equation 12 is practically the same as the calibration factor R calculated using the above-described Equation 8.

The value of $V_{0r}$ is calculated by the equation of $V_{0r} = g_{ref}(Th_{New})$ using the fourth calibration curve and the thermistor temperature $Th_{New}$ at the time of calibration. On the other hand, $V_{0r}'$ is a concentration output value in a case where the second band pass filter 36 is selected.

In a case where the calibration factor R is calculated, the concentration conversion processing circuit 46 calibrates the second calibration curve using the calculated calibration factor R. Specifically, the above-described Equation 9 is used as a post-calibration second calibration curve.

The timing of execution of the calibration processing has already been described in the first embodiment with reference to Figs. 7 and 8, and the detailed description thereof will not be repeated.

Thus, even in a case where the output characteristics of the gas concentration detection apparatus 10 change because of, for example, the deterioration of a component such as the light source 20, the gas concentration detection apparatus 10 according to this embodiment calculates the calibration factor R using the fourth calibration curve and the above-described Equation 12 and can appropriately calibrate the second calibration curve using the calculated calibration factor R. The deterioration in detection accuracy of a gas concentration can therefore be suppressed. It is therefore possible to provide a gas concentration detection apparatus that appropriately calibrates a changed output characteristics and a gas concentration calculation method for a gas concentration detection apparatus.

By calculating the calibration factor R using the fourth calibration curve, the calibration factor R with which the second calibration curve can be appropriately calibrated irrespective of a concentration at the time of calibration can be calculated.

It should be understood that the above-described embodiments are illustrative only and are not intended to limit the scope of the present invention. The scope of the present invention should be determined in view of the appended claims.

Reference Signs List

10 gas concentration detection apparatus
12 circuit board
14 cover
16 opening
18 optical path
20 light source
22 holding table
23 reflection member
24 pyroelectric sensor
28 thermistor
30 concentration detection unit
32 switcher
34 first band pass filter
36 second band pass filter
40 driving circuit
42 amplification circuit
44 A/D conversion circuit
46 concentration conversion processing circuit
48 switching driving circuit

**Claims**

1. A gas concentration calculation method for a gas concentration detection apparatus (10) that includes a light source (20) configured to emit infrared radiation, a light-receiving sensor (24) configured to receive the infrared radiation emitted from the light source and output a first output value corresponding to an amount of light received, a reflection member (23) that is provided between the light source (20) and the light-receiving sensor (24) and is configured to form an optical path (18) of the infrared radiation, and a filter (34, 36) configured to, on the optical path (18), select and pass one of a first wavelength band in which infrared radiation absorptance of detection target gas is higher than that in another wavelength band and a second wavelength band in which infrared radiation absorptance of detection target gas is lower than that in the first wavelength band, and a temperature sensor (28) configured to output a second output value corresponding to a temperature of the gas, the gas concentration calculation method comprising:

   a step of determining in advance the first output value obtained when the filter passes (34, 36) infrared radiation in the first wavelength band, a first calibration curve indicating a relationship between a concentration of the gas at a criterion temperature and a value obtained by normalizing the first output value with a criterion output value, and a second calibration curve indicating a relationship between the second output value in a criterion concentration and the criterion output value;
   a step of calculating a calibration factor used to calibrate the second calibration curve on the basis of the first output value obtained when the filter (34, 36) passes infrared radiation in the second wavelength band;
   a step of calibrating the second calibration curve on the basis of the calibration factor; and
   a step of calculating a concentration of the gas on the basis of the first calibration curve and the calibrated second calibration curve.

**2.** The gas concentration calculation method according to claim 1, wherein the calibration factor calculating step includes,
a step of calculating a conversion factor representing a ratio of a criterion output value, which is the first output value in the criterion concentration when the filter (34, 36) passes infrared radiation in the first wavelength band, to a reference output value, which is the first output value in the criterion concentration when the filter passes infrared radiation in the second wavelength band, and
a step of calculating the calibration factor on the basis of the second output value, the reference output value, and the conversion factor.

**3.** The gas concentration calculation method according to claim 2, wherein, in the conversion factor calculating step, the conversion factor corresponding to the second output value in the criterion concentration is calculated on the basis of a third calibration curve determined in advance.

**4.** The gas concentration calculation method according to claim 1, wherein the calibration factor calculating step includes,
a step of calculating a reference output value corresponding to the second output value obtained when the filter (34, 36) passes infrared radiation in the second wavelength band using a fourth calibration curve determined in advance, and
a step of calculating, as the calibration factor, a ratio of the reference output value to the first output value obtained when the filter passes infrared radiation in the second wavelength band.

**5.** A gas concentration detection apparatus (10) comprising:

a light source (20) configured to emit infrared radiation;
a light-receiving sensor (24) configured to receive the infrared radiation emitted from the light source (20) and output a first output value corresponding to an amount of light received;
a reflection member (23) that is provided between the light source (20) and the light-receiving sensor (24) and is configured to form an optical path (18) of the infrared radiation;
a filter (34, 36) configured to, on the optical path (18), select and pass one of a first wavelength band in which infrared radiation absorptance of detection target gas is higher than that in another wavelength band and a second wavelength band in which infrared radiation absorptance of detection target gas is lower than that in the first wavelength band;
a temperature sensor (28) configured to output a second output value corresponding to a temperature of the gas; and
a calculation unit (46) configured to calculate a concentration of gas on the optical path,
wherein the first output value obtained when the filter (34, 36) passes infrared radiation in the first wavelength band, a first calibration curve indicating a relationship between a concentration of the gas at a criterion temperature and a value obtained by normalizing the first output value with a criterion output value, and a second calibration curve indicating a relationship between the second output value in a criterion concentration and the criterion output value are determined in advance, and
wherein the calculation unit (46) calculates a calibration factor used to calibrate the second calibration curve on the basis of the first output value obtained when the filter (34, 36) passes infrared radiation in the second wavelength band, calibrates the second calibration curve on the basis of the calibration factor, and calculates a concentration of the gas on the basis of the first calibration curve and the calibrated second calibration curve.

**6.** The gas concentration detection apparatus according to claim 5, wherein the calculation unit (46) calculates a conversion factor representing a ratio of a criterion output value, which is the first output value in the criterion concentration when the filter passes infrared radiation in the first wavelength band, to a reference output value, which is the first output value in the criterion concentration when the filter passes infrared radiation in the second wavelength band, and calculates the calibration factor on the basis of the second output value, the reference output value, and the conversion factor.

**7.** The gas concentration detection apparatus according to claim 6, wherein the calculation unit (46) calculates the conversion factor corresponding to the second output value in the criterion concentration on the basis of a third calibration curve determined in advance.

**8.** The gas concentration detection apparatus according to claim 5, wherein the calculation unit (46) calculates a reference output value corresponding to the second output value obtained when the filter passes infrared radiation in the second wavelength band using a fourth calibration curve determined in advance, and calculates, as the calibration factor, a ratio of the reference output value to the first output value obtained when the filter passes infrared radiation in the second wavelength band.

9. The gas concentration detection apparatus according to any one of claims 5 to 8, wherein the calculation unit (46) calculates the calibration factor each time a period of time determined in advance has elapsed.

10. The gas concentration detection apparatus according to any one of claims 5 to 9, wherein the calculation unit (46) recalculates the calibration factor when a change in the second output value from a point in time when the calibration factor has been calculated is larger than or equal to a value determined in advance.

11. The gas concentration detection apparatus according to claim 10, wherein, when there is, in a history of previously calculated calibration factors, a calibration factor that satisfies conditions that the calibration factor has been obtained within a validity period of time and a difference between the second output value and the second output value obtained when the calibration factor has been calculated is smaller than a value determined in advance, the second calibration curve is calibrated using the calibration factor.

**Patentansprüche**

1. Gaskonzentrationsberechnungsverfahren für eine Gaskonzentrationserkennungsvorrichtung (10) mit einer Lichtquelle (20), die zum Abstrahlen von Infrarotstrahlung konfiguriert ist, einem Lichtempfangssensor (24), der zum Empfangen der von der Lichtquelle abgestrahlten Infrarotstrahlung und zum Ausgeben eines ersten Ausgangswerts, der einer empfangenen Lichtmenge entspricht, konfiguriert ist, einem Spiegelelement (23), das zwischen der Lichtquelle (20) und dem Lichtempfangssensor (24) bereitgestellt ist und zum Bilden eines optischen Wegs (18) der Infrarotstrahlung konfiguriert ist, und einem Filter (34, 36), das, auf dem optischen Weg, zum Auswählen und Durchlassen von einem von einem ersten Wellenlängenband, in dem der Infrarotstrahlungsabsorptionsgrad von Detektionszielgas höher als der in einem anderen Wellenlängenband ist, und einem zweiten Wellenlängenband, in dem der Infrarotstrahlungsabsorptionsgrad von Detektionszielgas niedriger als das im ersten Wellenlängenband ist, und einem Temperatursensor (28), der zum Ausgeben eines zweiten Ausgangswerts, der einer Temperatur des Gases entspricht, konfiguriert ist, wobei das Gaskonzentrationsberechnungsverfahren Folgendes aufweist:

einen Schritt des Voraus-Ermittelns des ersten Ausgangswerts, der erhalten wird, wenn das Filter (34, 36) Infrarotstrahlung im ersten Wellenlängenband durchlässt, einer ersten Kalibrationskurve, die eine Beziehung zwischen einer Konzentration des Gases bei einer Kriterium-Temperatur und einem Wert, der durch Normalisierung des ersten Ausgangswerts mit einem Kriterium-Ausgangswert erhalten wird, angibt, und einer zweiten Kalibrationskurve, die eine Beziehung zwischen dem zweiten Ausgangswert in einer Kriterium-Konzentration und dem Kriterium-Ausgangswert angibt;
einen Schritt des Berechnens eines Kalibrationsfaktors, der zum Kalibrieren der zweiten Kalibrationskurve auf Basis des ersten Ausgangswerts verwendet wird, der erhalten wird, wenn das Filter Infrarotstrahlung im zweiten Wellenlängenband durchlässt (34, 36);
einen Schritt des Kalibrierens der zweiten Kalibrationskurve auf Basis des Kalibrationsfaktors; und
einen Schritt des Berechnens einer Konzentration des Gases auf Basis der ersten Kalibrationskurve und der kalibrierten zweiten Kalibrationskurve.

2. Gaskonzentrationsberechnungsverfahren nach Anspruch 1, wobei der Kalibrationsfaktorberechnungsschritt Folgendes enthält:

einen Schritt des Berechnens eines Umwandlungsfaktors, der ein Verhältnis eines Kriterium-Ausgangswerts, welcher der erste Ausgangswert in der Kriterium-Konzentration ist, wenn das Filter (34, 36) Infrarotstrahlung im ersten Wellenlängenband durchlässt, zu einem Referenzausgangswert, der der erste Ausgangswert in der Kriterium-Konzentration ist, wenn das Filter Infrarotstrahlung im zweiten Wellenlängenband durchlässt, und einen Schritt des Berechnens des Kalibrierungsfaktors auf der Basis des zweiten Ausgangswerts, des Referenzausgangswerts und des Umwandlungsfaktors ist.

3. Gaskonzentrationsberechnungsverfahren nach Anspruch 2, wobei der dem zweiten Ausgangswert in der Kriterium-Konzentration entsprechende Umwandlungsfaktor im Umwandlungsfaktorberechnungsschritt auf Basis einer im Voraus ermittelten dritten Kalibrationskurve berechnet wird.

4. Gaskonzentrationsberechnungsverfahren nach Anspruch 1, wobei der Kalibrationsfaktorberechnungsschritt Folgendes enthält:

   einen Schritt des Berechnens eines Referenzausgangswerts, der dem zweiten Ausgangswert entspricht, der erhalten wird, wenn das Filter (34, 36) Infrarotstrahlung im zweiten Wellenlängenband durchlässt, unter Verwendung einer vierten im Voraus ermittelten Kalibrationskurve, und

   einen Schritt des Berechnens eines Verhältnisses des Referenzausgangswerts zu dem ersten Ausgangswert, der erhalten wird, wenn das Filter Infrarotstrahlung im zweiten Wellenlängenband durchlässt, als den Kalibrationsfaktor.

5. Gaskonzentrationserkennungsvorrichtung (10), die Folgendes aufweist:

   eine Lichtquelle (20), die zum Abstrahlen von Infrarotstrahlung konfiguriert ist;

   einen Lichtempfangssensor (24), der zum Empfangen der von der Lichtquelle (20) abgestrahlten Infrarotstrahlung und zum Ausgeben eines ersten Ausgangswerts, der einer empfangenen Lichtmenge entspricht, konfiguriert ist;

   ein Spiegelelement (23), das zwischen der Lichtquelle (20) und dem Lichtempfangssensor (24) bereitgestellt ist und zum Bilden eines optischen Wegs (18) der Infrarotstrahlung konfiguriert ist;

   und ein Filter (34, 36), das, auf dem optischen Weg, zum Auswählen und Durchlassen von einem von einem ersten Wellenlängenband, in dem der Infrarotstrahlungsabsorptionsgrad von Detektionszielgas höher als der in einem anderen Wellenlängenband ist, und einem zweiten Wellenlängenband, in dem der Infrarotstrahlungsabsorptionsgrad von Detektionszielgas niedriger als das im ersten Wellenlängenband ist,;

   einen Temperatursensor (28), der zum Ausgeben eines zweiten Ausgangswerts, der einer Temperatur des Gases entspricht, konfiguriert ist; und

   eine Berechnungseinheit (46), die zum Berechnen einer Gaskonzentration auf dem optischen Weg konfiguriert ist,

   wobei der ersten Ausgangswert, der erhalten wird, wenn das Filter (34, 36) Infrarotstrahlung im zweiten Wellenlängenband durchlässt, eine erste Kalibrationskurve, die eine Beziehung zwischen einer Konzentration des Gases bei einer Kriterium-Temperatur und einem Wert, der durch Normalisierung des ersten Ausgangswerts mit einem Kriterium-Ausgangswert erhalten wird, angibt, und eine zweite Kalibrationskurve, die eine Beziehung zwischen dem zweiten Ausgangswert in einer Kriterium-Konzentration und dem Kriterium-Ausgangswert angibt, im Voraus ermittelt werden, und

   wobei die Berechnungseinheit (46) einen Kalibrationsfaktor berechnet, der zum Kalibrieren der zweiten Kalibrationskurve auf Basis des ersten Ausgangswerts verwendet wird, der erhalten wird, wenn das Filter (34, 36) Infrarotstrahlung im zweiten Wellenlängenband durchlässt, die zweiten Kalibrationskurve auf Basis des Kalibrationsfaktors berechnet und eine Konzentration des Gases auf Basis der ersten Kalibrationskurve und der kalibrierten zweiten Kalibrationskurve berechnet.

6. Gaskonzentrationserkennungsvorrichtung nach Anspruch 5, wobei die Berechnungseinheit (46) einen Umwandlungsfaktor berechnet, der ein Verhältnis eines Kriterium-Ausgangswerts, welcher der erste Ausgangswert in der Kriterium-Konzentration ist, wenn das Filter Infrarotstrahlung im ersten Wellenlängenband durchlässt, zu einem Referenzausgangswert, der der erste Ausgangswert in der Kriterium-Konzentration ist, wenn das Filter Infrarotstrahlung im zweiten Wellenlängenband durchlässt, und den Kalibrierungsfaktor auf der Basis des zweiten Ausgangswerts, des Referenzausgangswerts und des Umwandlungsfaktors ist, berechnet.

7. Gaskonzentrationserkennungsvorrichtung nach Anspruch 6, wobei die Berechnungseinheit (46) den dem zweiten Ausgangswert in der Kriterium-Konzentration entsprechenden Umwandlungsfaktor auf Basis einer im Voraus ermittelten dritten Kalibrationskurve berechnet.

8. Gaskonzentrationserkennungsvorrichtung nach Anspruch 5, wobei die Berechnungseinheit (46) einen Referenzausgangswert, der dem zweiten Ausgangswert entspricht, der erhalten wird, wenn das Filter Infrarotstrahlung im zweiten Wellenlängenband durchlässt, unter Verwendung einer vierten im Voraus ermittelten Kalibrationskurve berechnet und als den Kalibrationsfaktor ein Verhältnis des Referenzausgangswerts zu dem ersten Ausgangswert, der erhalten wird, wenn das Filter Infrarotstrahlung im zweiten Wellenlängenband durchlässt berechnet.

9. Gaskonzentrationserkennungsvorrichtung nach einem der Ansprüche 5 bis 8, wobei die Berechnungseinheit (46) den Kalibrationsfaktor jedesmal berechnet, wenn eine im Voraus bestimmte Zeitspanne verstrichen ist.

**10.** Gaskonzentrationserkennungsvorrichtung nach einem der Ansprüche 5 bis 9, wobei die Berechnungseinheit (46) den Kalibrationsfaktor erneut berechnet, wenn eine Änderung des zweiten Ausgangswerts gegenüber einem Zeitpunkt, an dem der Kalibrationsfaktor berechnet wurde, so groß wie ein im Voraus ermittelter Wert oder größer ist.

**11.** Gaskonzentrationserkennungsvorrichtung nach Anspruch 10, wobei in einer Historie zuvor berechneter Kalibrationsfaktoren, falls es sie gibt, ein Kalibrationsfaktor, der Bedingungen erfüllt, dass der Kalibrationsfaktor innerhalb einer Gültigkeitsdauer erhalten wurde und eine Differenz zwischen dem zweiten Ausgangswert und dem zweiten Ausgangswert, der erhalten wird, wenn der Kalibrationsfaktor berechnet wurde, kleiner als ein im Voraus ermittelter Wert ist, die zweite Kalibrationskurve unter Verwendung des Kalibrationsfaktors kalibriert wird.

**Revendications**

**1.** Procédé de calcul de concentration de gaz pour un appareil de détection de concentration de gaz (10) qui comprend une source lumineuse (20) configurée pour émettre un rayonnement infrarouge, un capteur de réception de lumière (24) configuré pour recevoir le rayonnement infrarouge émis de la source lumineuse et sortir une première valeur de sortie correspondant à une quantité de lumière reçue, un membre de réflexion (23) qui est fourni entre la source lumineuse (20) et le capteur de réception de lumière (24) et est configuré pour former un chemin optique (18) du rayonnement infrarouge, et un filtre (34, 36) configuré pour, sur le chemin optique (18), sélectionner et passer l'une d'entre une première bande de longueur d'onde dans laquelle l'absorptance du rayonnement infrarouge du gaz cible de détection est plus élevée que celle dans une autre bande de longueur d'onde et une deuxième bande de longueur d'onde dans laquelle l'absorptance du rayonnement infrarouge du gaz cible de détection est plus basse que celle dans la première bande de longueur d'onde, et un capteur de température (28) configuré pour sortir une deuxième valeur de sortie correspondant à une température du gaz, la méthode de calcul de concentration de gaz comprenant :

une étape consistant à déterminer à l'avance la première valeur de sortie obtenue lorsque le filtre (34, 36) passe un rayonnement infrarouge dans la première bande de longueur d'onde, une première courbe d'étalonnage indiquant une relation entre une concentration du gaz à une température critère et une valeur obtenue en normalisant la première valeur de sortie avec une valeur de sortie critère, et une deuxième courbe d'étalonnage indiquant une relation entre la deuxième valeur de sortie dans une concentration critère et la valeur de sortie critère ;
une étape consistant à calculer un facteur d'étalonnage utilisé pour étalonner la deuxième courbe d'étalonnage sur la base de la première valeur de sortie obtenue lorsque le filtre (34, 36) passe un rayonnement infrarouge dans la deuxième bande de longueur d'onde ;
une étape consistant à étalonner la deuxième courbe d'étalonnage sur la base du facteur d'étalonnage ; et
une étape consistant à calculer une concentration du gaz sur la base de la première courbe d'étalonnage et de la deuxième courbe d'étalonnage étalonnée.

**2.** Procédé de calcul de concentration de gaz selon la revendication 1, dans lequel l'étape de calcul du facteur d'étalonnage comprend :

une étape consistant à calculer un facteur de conversion représentant un rapport d'une valeur de sortie critère, qui est la première valeur de sortie dans la concentration critère lorsque le filtre (34, 36) passe un rayonnement infrarouge dans la première bande de longueur d'onde, à une valeur de sortie de référence, qui est la première valeur de sortie dans la concentration critère lorsque le filtre passe un rayonnement infrarouge dans la deuxième bande de longueur d'onde, et
une étape consistant à calculer le facteur d'étalonnage sur la base de la deuxième valeur de sortie, de la valeur de sortie de référence et du facteur de conversion.

**3.** Procédé de calcul de concentration de gaz selon la revendication 2, dans lequel à l'étape de calcul du facteur de conversion, le facteur de conversion correspondant à la deuxième valeur de sortie dans la concentration critère est calculé sur la base d'une troisième courbe d'étalonnage déterminée à l'avance.

**4.** Procédé de calcul de concentration de gaz selon la revendication 1, dans lequel l'étape de calcul du facteur d'étalonnage comprend :

une étape consistant à calculer une valeur de sortie de référence correspondant à la deuxième valeur de sortie obtenue lorsque le filtre (34, 36) passe un rayonnement infrarouge dans la deuxième bande de longueur d'onde en utilisant une quatrième courbe d'étalonnage déterminée à l'avance, et

une étape consistant à calculer, en tant que facteur d'étalonnage, un rapport de la valeur de sortie de référence à la première valeur de sortie obtenue lorsque le filtre passe un rayonnement infrarouge dans la deuxième bande de longueur d'onde.

5. Appareil de détection de concentration de gaz (10) comprenant :

une source lumineuse (20) configurée pour émettre un rayonnement infrarouge ;
un capteur de réception de lumière (24) configuré pour recevoir le rayonnement infrarouge émis de la source lumineuse (20) et sortir une première valeur de sortie correspondant à une quantité de lumière reçue ;
un membre de réflexion (23) qui est fourni entre la source lumineuse (20) et le capteur de réception de lumière (24) et est configuré pour former un chemin optique (18) du rayonnement infrarouge ;
un filtre (34, 36) configuré pour, sur le chemin optique (18), sélectionner et passer l'une d'entre une première bande de longueur d'onde dans laquelle l'absorptance de rayonnement infrarouge de gaz cible de détection est plus élevée que celle dans une autre bande de longueur d'onde et une deuxième bande de longueur d'onde dans laquelle l'absorptance de rayonnement infrarouge de gaz cible de détection est plus basse que celle dans la première bande de longueur d'onde ;
un capteur de température (28) configuré pour sortir une deuxième valeur de sortie correspondant à une température du gaz ; et
une unité de calcul (46) configurée pour calculer une concentration de gaz sur le chemin optique,
dans lequel la première valeur de sortie obtenue lorsque le filtre (34, 36) passe un rayonnement infrarouge dans la première bande de longueur d'onde, une première courbe d'étalonnage indiquant une relation entre une concentration du gaz à une température critère et une valeur obtenue en normalisant la première valeur de sortie avec une valeur de sortie critère, et une deuxième courbe d'étalonnage indiquant une relation entre la deuxième valeur de sortie dans une concentration critère et la valeur de sortie critère sont déterminées à l'avance, et
dans lequel l'unité de calcul (46) calcule un facteur d'étalonnage utilisé pour étalonner la deuxième courbe d'étalonnage sur la base de la première valeur de sortie obtenue lorsque le filtre (34, 36) passe un rayonnement infrarouge dans la deuxième bande de longueur d'onde, étalonne la deuxième courbe d'étalonnage sur la base du facteur d'étalonnage, et calcule une concentration du gaz sur la base de la première courbe d'étalonnage et de la deuxième courbe d'étalonnage.

6. Appareil de détection de concentration de gaz selon la revendication 5, dans lequel l'unité de calcul (46) calcule un facteur de conversion représentant un rapport d'une valeur de sortie critère, qui est la première valeur de sortie dans la concentration critère lorsque le filtre passe un rayonnement infrarouge dans la première bande de longueur d'onde, à une valeur de sortie de référence, qui est la première valeur de sortie dans la concentration critère lorsque le filtre passe un rayonnement infrarouge dans la deuxième bande de longueur d'onde, et calcule le facteur d'étalonnage sur la base de la deuxième valeur de sortie, de la valeur de sortie de référence et du facteur de conversion.

7. Appareil de détection de concentration de gaz selon la revendication 6, dans lequel l'unité de calcul (46) calcule le facteur de conversion correspondant à la deuxième valeur de sortie dans la concentration critère sur la base d'une troisième courbe d'étalonnage déterminée à l'avance.

8. Appareil de détection de concentration de gaz selon la revendication 5, dans lequel l'unité de calcul (46) calcule une valeur de sortie de référence correspondant à la deuxième valeur de sortie obtenue lorsque le filtre passe un rayonnement infrarouge dans la deuxième bande de longueur d'onde en utilisant une quatrième courbe d'étalonnage déterminée à l'avance, et calcule, en tant que facteur d'étalonnage, un rapport de la valeur de sortie de référence à la première valeur de sortie obtenue lorsque le filtre passe un rayonnement infrarouge dans la deuxième bande de longueur d'onde.

9. Appareil de détection de concentration de gaz selon l'une quelconque des revendications 5 à 8, dans lequel l'unité de calcul (46) calcule le facteur d'étalonnage chaque fois qu'une période de temps déterminée à l'avance s'est écoulée.

10. Appareil de détection de concentration de gaz selon l'une quelconque des revendications 5 à 9, dans lequel l'unité de calcul (46) recalcule le facteur d'étalonnage lorsqu'un changement dans la deuxième valeur de sortie d'un point dans le temps lorsque le facteur d'étalonnage a été calculé, est plus grand ou égal à une valeur déterminée à l'avance.

11. Appareil de détection de concentration de gaz selon la revendication 10, dans lequel, lorsqu'il y a, dans un historique de facteurs d'étalonnage calculés précédemment, un facteur d'étalonnage qui satisfait les conditions que le facteur d'étalonnage a été obtenu dans une période de validité de temps et qu'une différence entre la deuxième valeur de sortie et la deuxième valeur de sortie obtenue lorsque le facteur d'étalonnage a été calculé est plus petite qu'une valeur déterminée à l'avance, la deuxième courbe d'étalonnage est étalonnée en utilisant le facteur d'étalonnage.

FIG.1

FIG.2

FIG.3

FIG.4

| TEMPERATURE [°C] CONCENTRATION [ppm] | 0 | 10 | 25 (CRITERION) | 40 | 50 | |
|---|---|---|---|---|---|---|
| 0 (CRITERION) | O | O | O | O | O | SECOND CALIBRATION CURVE THIRD CALIBRATION CURVE |
| 400 | | | O | | | |
| 1000 | | | O | | | |
| 2000 | | | O | | | |

FIRST CALIBRATION CURVE

FIG.5

FIG.6

CONVERSION
FACTOR C

TEMPERATURE [°C]

FIG.7

```
                    ┌──────────┐
                    │  START   │
                    └────┬─────┘
                         │
                         ▼           ┌S100
              ◇ HAS FIRST CALIBRATION ◇────NO──────────┐
              │ PROCESSING ALREADY    │                │
              │ BEEN PERFORMED?       │                ▼        ┌S102
                         │            ◇ ARE CONDITIONS FOR      ◇────NO──────┐
                        YES           │ EXECUTION OF FIRST      │            │
                         │            │ CALIBRATION PROCESSING  │            │
                         │            │ SATISFIED?              │            │
                         │                     │                             │
                         │                    YES    ┌S104                   │
                         │            ┌─────────────────────┐                │
                         │            │ PERFORM FIRST CALIBRATION │          │
                         │            │ PROCESSING          │                │
                         │            └──────────┬──────────┘                │
                         │◄──────────────────────┘                          │
                         │                                                    │
                         ▼          ┌S106                                    │
              ◇ IS TIME CONDITION   ◇────NO──────────┐                       │
              │ SATISFIED?          │                │                       │
                         │                           ▼          ┌S108        │
                        YES                ◇ IS TEMPERATURE      ◇───NO───────┤
                         │                 │ CHANGE CONDITION    │            │
                         │                 │ SATISFIED?          │            │
                         │                          │                         │
                         │                         YES   ┌S110               │
                         │◄──────NO──── ◇ IS THERE AVAILABLE ◇                │
                         │              │ CALIBRATION FACTOR   │               │
                         │              │ HISTORY?             │               │
                         │                       │                            │
                         │                      YES   ┌S114                   │
                         ▼  ┌S112                 ▼                           │
              ┌─────────────────────┐  ┌─────────────────────┐               │
              │ PERFORM CALIBRATION │  │ UPDATE CALIBRATION FACTOR │          │
              │ PROCESSING          │  └──────────┬──────────┘               │
              └──────────┬──────────┘             │                          │
                         │                        │◄─────────────────────────┘
                         ▼                        │
                    ┌──────────┐                  │
                    │  RETURN  │◄─────────────────┘
                    └──────────┘
```

FIG.8

EP 3 179 233 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013076634 A